# EUROPEAN PATENT SPECIFICATION

(11) **EP 3 472 624 B1**
(45) Date of publication and mention of the grant of the patent: **04.11.2020**
(21) Application number: 17732835.8
(22) Date of filing: 14.06.2017
(51) Int. Cl.: G01N 33/86

(54) **IMPROVED D-DIMER ASSAY CALIBRATION**
VERBESSERTE KALIBRIERUNG EINES D-DIMER-TESTS
ÉTALONNAGE DE DOSAGE D-DIMÈRE AMÉLIORÉ

(30) Priority: 15.06.2016 EP 16174523
(43) Date of publication of application: 24.04.2019
(73) Proprietor: Roche Diagnostics GmbH, 68305 Mannheim (DE); F. Hoffmann-La Roche AG, 4070 Basel (CH)
(72) Inventor: GOTTSCHALK, Norbert, 82362 Weilheim (DE); JAGDHUBER, Franziska, 82110 Germering (DE); TOWN, Michael-Harold, 82386 Oberhausen (DE)
(74) Representative: Altmann Stößel Dick Patentanwälte PartG mbB
(86) International application number: PCT/EP2017/064500
(87) International publication number: WO 2017/216208

(56) References cited:
- US-B2- 8 900 872
- PASSING H ET AL: "A new biometrical procedure for testing the equality of measurements from two different analytical methods: application of linear regression procedures for method comparison studies in clinical chemistry, part I", JOURNAL OF CLINICAL CHEMISTRY & CLINICAL BIOCHEMISTRY, BERLIN, DE, vol. 21, no. 11, 1 January 1983 (1983-01-01), pages 709-720, XP008118228,
- JENNINGS I ET AL: "Laboratory D-dimer measurement: Improved agreement between methods through calibration", THROMBOSIS AND HAEMOSTASIS, SCHATTAUER GMBH, DE , vol. 98, no. 5 1 November 2007 (2007-11-01), pages 1127-1135, XP008182198, ISSN: 0340-6245 Retrieved from the Internet: URL:https://th.schattauer.de/en/contents/a rchive/issue/742/manuscript/8857/download. html [retrieved on 2007-10-11]
- NIEUWENHUIZEN WILLEM: "A reference material for harmonisation of D-dimer assays", THROMBOSIS AND HAEMOSTASIS, SCHATTAUER GMBH, DE, vol. 77, no. 5, 1 January 1997 (1997-01-01), pages 1031-1033, XP008182199, ISSN: 0340-6245

## Description

The present invention relates to a method for providing a calibration curve for an optical D-dimer assay comprising a) providing a calibrator stock solution having a predetermined D-dimer concentration, b) providing at least two calibrator samples, wherein said at least two calibrator samples are derived from said calibrator stock solution, c) determining the value of an optical property of said calibrator samples of b) in said D-dimer assay, d) assigning an assigned concentration value to at least one calibrator sample of b), wherein said assigned concentration value assigned to at least one calibrator sample deviates from the calculated concentration value of said calibrator sample, and e) establishing a calibration curve based on the values of said optical property of c), on the assigned concentration value or the assigned concentration values of d), and on calculated concentration values for calibrator samples to which no assigned concentration value was assigned. The present invention further relates to a method for determining the D-dimer concentration in a sample based on said calibration curve. Further disclosure relates to kits, devices, databases, data carriers, and uses related thereto.

The D-dimer assay is of clinical significance, since increased D-dimer concentrations in a sample of a subject indicate the presence of clotted blood which is being degraded and, thus, may indicate severe conditions including thrombosis, embolism, coagulopathy, and the like. In clinical practice, the D-dimer assay is used to provide an exclusion diagnosis of lung embolism and deep vein thrombosis, i.e. a D-dimer concentration below a pre-determined cutoff value is indicative of a subject not suffering from lung embolism and not suffering from deep vein thrombosis (Ramzi & Leeper (2004), Am. Fam. Phys 69(12):2829; Schutgens et al. (2003), Circulation 2003;107:593-597). The D-dimer assay most frequently used is an immuno turbidimetry assay using latex beads coated with anti-D-dimer antibodies (cf. e.g. US 8,900,872 B2). Dependent on the concentration of D-dimers in the sample, formation of D-dimer/antibody complexes induces agglutination of the latex beads and induces an increase in turbidity of the reaction mixture proportional to the D-dimer concentration.

Clinical assays for coagulation parameters, in particular immunological assays, generally require performing a calibration, i.e. establishing a relationship between the measured parameter, e.g. a photometrically determined signal, and the parameter to be determined, e.g. a concentration of an analyte. The calibration is usually performed by determining the measured parameter on a set of samples having a known, predetermined concentration of the analyte, and establishing a calibration curve. Depending on the kind of assay, a more or less pronounced dependency of the calibration results on the device used, the specific batch of reagents, and even the day the calibration is performed may be observed, such that it is common practice to establish a complete calibration curve for each measurement. However, such proceeding is labor and cost intensive. Therefore, it was attempted to simplify calibration.

In US 2007/0020765 A1, calibration of an activated partial thromboplastin time (APTT) assay was performed with only two calibrator samples; this proceeding, however, requires that the calibration curve is linear. In a different approach, a single calibration adjuster was used to calculate a corrected value of the measured parameter for each sample in several blood clotting assays (US 8,900,872 B2). However, this approach requires that the deviations follow the mathematical formula assumed to underlie the deviations. Passing et al. (1983), J Clin Chem & Clin Biochem 21(11):709 teach procedures for statistical evaluation of method comparisons and instrument tests. Jennings et al (2007), Thrombosis and Hemostasis 98(5), 127 relates to improvement of agreement of D-dimer measurements between different laboratories through calibration. Nieuwenhuizen et al. (1997), Thrombosis and Hemostasis 77(5):1031 proposed a reference material for harmonization of D-Dimer assays.

There is, thus, a need in the art for improved means and methods for calibrating coagulation assays, avoiding the drawbacks of the prior art. This problem is solved by the means and methods disclosed herein.

Accordingly, the present invention relates to a method for providing a calibration curve for an optical D-dimer assay according to claims 1; further disclosure relates to a method for providing a calibration curve for an optical D-dimer assay comprising
a) providing a calibrator stock solution having a predetermined D-dimer concentration,
b) providing at least two calibrator samples, wherein said at least two calibrator samples are derived from said calibrator stock solution,
c) determining the value of an optical property of said calibrator samples of b) in said D-dimer assay,
d) assigning an assigned concentration value to at least one calibrator sample of b), wherein said assigned concentration value assigned to at least one calibrator sample deviates from the calculated concentration value of said calibrator sample, and
e) establishing a calibration curve based on the values of said optical property of c), on the assigned concentration value or the assigned concentration values of d), and on calculated concentration values for calibrator samples to which no assigned concentration value was assigned.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, as used in the following, the terms "preferably", "more preferably", "most preferably", "particularly", "more particularly", "specifically", "more specifically" or similar terms are used in conjunction with optional features, without restricting further possibilities. Thus, features introduced by these terms are optional features and are not intended to restrict the scope of the claims in any way. Similarly, features introduced by "in an embodiment of the invention" or similar expressions are intended to be optional features, without any restriction regarding further embodiments of the invention, without any restrictions regarding the scope of the invention and without any restriction regarding the possibility of combining the features introduced in such way with other optional or non-optional features of the invention. Moreover, if not otherwise indicated, the term "about" relates to the indicated value with the commonly accepted technical precision in the relevant field, preferably relates to the indicated value ± 20%, more preferably ± 10%, most preferably ± 5%.

The methods of the present invention are in vitro methods. Moreover, they may comprise steps in addition to those explicitly mentioned. For example, the method for providing a calibration curve may comprise the further steps of contacting calibrator samples with a detection agent specifically binding to D-dimers in the D-dimer assay of step c), and/or providing the calibration curve established in step e), e.g. to an evaluation unit of a device of the present invention. Moreover, one or more steps of the methods of the present invention may be assisted or performed by automated equipment.

The term "D-dimer", as used herein, relates to a polypeptide comprising two cross-linked fibrin D-domains, generated by plasmin-catalyzed hydrolysis of Fibrin. In an embodiment, the term relates to human D-dimer. The D-dimer molecule is known to the skilled person, as are, in principle, methods for its determination (Dempfle CE (2005), Semin Vasc Med 5:315-320; EP1695984A1, Example 1). Accordingly, the term "D-dimer assay" relates to an assay determining the amount or concentration of D-dimers in a sample. In an embodiment, in the D-dimer assay, the concentration of D-dimers in a sample is determined. In an embodiment, the D-dimer assay is an optical assay, in a further embodiment is an assay comprising determining an optical property of a sample. As will be understood by the skilled person, in an embodiment, said optical property is determined at least once after contacting said sample with a detection agent; in a further embodiment, said optical property is determined before and after contacting said sample with a detection agent, and, in an embodiment, determining the amount or concentration of D-dimers in said sample is based on the difference between the value of said optical property determined after contacting said sample with a detection agent and the value of said optical property determined before contacting said sample with a detection agent. In an embodiment, the term D-dimer includes D-dimers which are hydrolysis products of splice variants of Fibrin and/or degradation products of Fibrin which contain D-dimer.

In an embodiment, the D-dimer assay comprises contacting calibrator samples with a detection agent specifically recognizing D-dimers. As the skilled artisan will appreciate, the terms "specifically recognizing" and "binding specifically", or grammatical variations thereof, are used to indicate that other compounds, typically biomolecules, present in a sample do not significantly bind to a ligand, in particular to a detection agent specifically recognizing D-dimers, of the present disclosure; in an embodiment, this does not exclude binding of chemical compounds, e.g. interfering compounds, to regions of the agents not involved in interaction with D-dimers. In an embodiment, the level of binding of a detection agent specifically binding to a compound other than a D-dimer results in a binding affinity which is at most 10%, at most 1%, at most 0.1 %, or at most 0.01% of the affinity to D-dimers. In an embodiment, the detection agent specifically recognizing D-dimers is a biological macromolecule, e.g. an aptamer. The detection agent specifically recognizing D-dimers has the biological activity of binding specifically to D-dimers. In an embodiment, a detection agent specifically recognizing D-dimers has at least two domains specifically binding to D-Dimers; thus, the detection agent specifically recognizing D-dimers, in an embodiment, is a D-dimer cross-linking agent. In an embodiment, the agent specifically recognizing D-dimers specifically binds to at least one epitope comprised in a D-dimer, wherein said epitope is an epitope specific for the D-dimer. In particular, said epitope may be an epitope becoming accessibly only after plasmin-catalyzed hydrolysis of Fibrin; thus, the epitope may also be comprised in a derivative of the D-dimer, wherein said derivative of a D-dimer is a compound correlating in amount with the D-dimer, e.g. is a degradation product of the D-dimer. In an embodiment, the detection agent specifically recognizing D-dimers is an antibody, in an embodiment a monoclonal antibody, in a further embodiment an F(ab)₂ fragment of a monoclonal antibody.

The term "antibody", as used herein, includes monoclonal antibodies, multispecific antibodies (e.g. bispecific antibodies) formed from at least two intact antibodies, and antibody fragments so long as they exhibit the desired binding activity as specified elsewhere herein. Thus, also multispecific antibodies according to the present disclosure bind specifically to D-dimers, e.g. by binding to two or more epitopes of a D-dimer. In an embodiment, the antibody is a monoclonal antibody. In an embodiment, the antibody is a full-length antibody or an antibody fragment.

Depending on the amino acid sequences of the constant domains of their heavy chains, antibodies (immunoglobulins) can be assigned to different classes. There are five major classes of immunoglobulins: IgA, IgD, IgE, IgG, and IgM, and several of these may be further divided into subclasses (isotypes), e.g., IgG1, IgG2, IgG3, IgG4, IgA1, and IgA2. The subunit structures and three-dimensional configurations of different classes of immunoglobulins are well known and described generally in, for example, Abbas et al., Cellular and Mol. Immunology, 4th ed., W.B. Saunders, Co. (2000). An antibody may be part of a larger fusion molecule, formed by covalent or non-covalent association of the antibody with one or more other proteins or peptides, dye, polymer, or the like.

The terms "full-length antibody," "intact antibody," and "whole antibody" are used herein interchangeably to refer to an antibody in its substantially intact form, not antibody fragments as defined below. The terms particularly refer to an antibody with heavy chains that contain an Fc region. "Antibody fragments" comprise a portion of an intact antibody, in an embodiment, comprising the antigen-binding region thereof. Examples of antibody fragments include Fab, Fab', F(ab)₂, and Fv fragments; diabodies; linear antibodies; single-chain antibody molecules, nanobodies, and multispecific antibodies formed from antibody fragments. Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab)₂ fragment that has two antigen-combining sites and is still capable of cross-linking antigen. "Fv" is the minimum antibody fragment which contains a complete antigen-binding site. In one embodiment, a two-chain Fv species consists of a dimer of one heavy- and one light-chain variable domain in tight, non-covalent association. In a single-chain Fv (scFv) species, one heavy- and one light-chain variable domain can be covalently linked by a flexible peptide linker such that the light and heavy chains can associate in a "dimeric" structure analogous to that in a two-chain Fv species. It is in this configuration that the three hypervariable regions (HVRs) of each variable domain interact to define an antigen-binding site. Collectively, the six HVRs confer antigen-binding specificity to the antibody. However, even a single variable domain (or half of an Fv comprising only three HVRs specific for an antigen) has the ability to recognize and bind antigen, although at a lower affinity than the entire binding site. The term "diabodies" refers to antibody fragments with two antigen-binding sites, which fragments comprise a heavy-chain variable domain (VH) connected to a light-chain variable domain (VL) in the same polypeptide chain (VH-VL). By using a linker that is too short to allow pairing between the two domains on the same chain, the domains are forced to pair with the complementary domains of another chain and create two antigen-binding sites. Diabodies may be bivalent or bispecific. Diabodies are described more fully in, for example, EP 0 404 097; WO 1993/01161; Hudson et al., Nat. Med. 9 (2003) 129-134; and Hollinger et al., PNAS USA 90 (1993) 6444-6448. Triabodies and tetrabodies are also described in Hudson et al., Nat. Med. 9 (2003) 129-134.

The term "monoclonal antibody" as used herein refers to an antibody obtained from a population of substantially homogeneous antibodies, i.e., the individual antibodies comprised in the population are identical except for possible mutations, e.g., naturally occurring mutations, that may be present in minor amounts. Thus, the modifier "monoclonal" indicates the character of the antibody as not being a mixture of discrete antibodies. In certain embodiments, such a monoclonal antibody typically includes an antibody comprising a polypeptide sequence that binds an analyte, wherein the analyte-binding polypeptide sequence was obtained by a process that includes the selection of a single analyte binding polypeptide sequence from a plurality of polypeptide sequences. For example, the selection process can be the selection of a unique clone from a plurality of clones, such as a pool of hybridoma clones, phage clones, or recombinant DNA clones. It should be understood that a selected target binding sequence can be further altered, for example, to improve affinity for the target, to humanize the target-binding sequence, to improve its production in cell culture, to reduce its immunogenicity in vivo, to create a multispecific antibody, etc., and that an antibody comprising the altered target binding sequence is also a monoclonal antibody of this disclosure. In contrast to polyclonal antibody preparations, which include different antibodies directed against different determinants (epitopes), each monoclonal antibody of a monoclonal-antibody preparation is directed against a single determinant on an antigen. In addition to their specificity, monoclonal-antibody preparations are advantageous in that they are typically uncontaminated by other immunoglobulins.

Antibodies or fragments thereof can be obtained by using methods which are described, e.g., in Harlow and Lane "Antibodies, A Laboratory Manual", CSH Press, Cold Spring Harbor, 1988. Monoclonal antibodies can be prepared by the techniques originally described in Kohler and Milstein, Nature 256 (1975), 495, and Galfré, Meth. Enzymol. 73 (1981), 3, which comprise the fusion of mouse myeloma cells to spleen cells derived from immunized mammals.

In an embodiment, the detection agent specifically recognizing D-dimers is covalently coupled to latex particles, i.e. to colloidal-dispersed polymer particles. In another embodiment the detection agent specifically recognizing D-dimers is attached to latex particles in a non-covalent way via bioaffine pairs such as biotin/avidin (or streptavidin). Suitable latex particles and suitable sizes thereof are known to the skilled person. The latex particles may be heterogeneous in size, e.g. in a size range of from 0.1 µm to 1 µm, in an embodiment of from 0.15 µm to 0.50 µm. In an embodiment, the latex particles are uniform in size.

The term "calibrator stock solution", as used herein, relates to a solution, in an embodiment an aqueous solution, of D-dimers having a known, in an embodiment pre-determined, concentration of D-dimers. As will be understood by the skilled person, in an embodiment, it is, in principle, possible to provide a known amount of D-dimers, e.g. of recombinant human D-dimer, and to dissolve said known amount in a known volume of liquid, thus providing a calibrator stock solution having a known concentration of D-dimers. In an embodiment, the calibrator stock solution is provided by mixing samples known to comprise D-dimers at appropriate concentrations and by calculating or, in an embodiment, by determining the D-dimer concentration in said mixture by a suitable assay. In an embodiment, the calibrator stock solution comprises D-dimers in a concentration corresponding to or exceeding the highest D-dimer concentration determined during calibration.

According to the invention, the number of calibrator stock solutions provided is lower than the number of calibrator samples used for establishing the calibration curve of the present invention. In an embodiment, one single, i.e. exactly one, calibrator stock solution is provided for establishing the calibration curve of the present invention. In a further embodiment, one single calibrator stock solution is provided for establishing the calibration curve of the present invention and all or all further calibration samples are derived from said single calibrator stock solution by dilution.

As used herein, the term "predetermined D-dimer concentration" relates to a D-dimer concentration known at the time the calibration curve of the present invention is established, e.g. a D-dimer concentration having been determined before the method of the present invention is performed. In an embodiment, the predetermined D-dimer concentration is the actual D-dimer concentration in the calibrator stock solution, wherein the actual D-dimer concentration is the D-dimer concentration as determinable according to Adema & Gebert (1995), Thromb Res 80:85. However, it is also envisaged that the predetermined D-dimer concentration is a D-dimer concentration determined on a different device, in an embodiment, on a different model of device. In an embodiment, the predetermined D-dimer concentration is a device-specific value, i.e. is a value assigned to said calibrator stock solution such that correct D-dimer concentrations are determined. Thus, in an embodiment, the predetermined D-dimer concentration may be an assigned value as specified herein. In a further embodiment, the predetermined D-dimer concentration corresponds to the D-dimer concentration as determinable according to Adema & Gebert ± 50 %, in an embodiment ± 30%.

The term "calibrator sample" as used herein, relates to a sample derived from the calibrator stock solution. Thus, in an embodiment, a calibrator solution having a D-dimer concentration of zero is not a calibrator sample according to the present invention. The calibrator sample is "derived" from the calibrator stock solution such that, based on the known D-dimer concentration of the calibrator stock solution, a calculated D-dimer concentration value can be attributed to said calibrator sample by mathematical means, in an embodiment by a basic arithmetic operation. Thus, in an embodiment, the calibrator sample may be derived from the calibrator stock solution by drying a known volume of said calibrator stock solution onto a solid surface and dissolving the residue in a known volume of liquid. In an embodiment, the calibrator sample is derived from the calibrator stock solution by dilution, in particular by mixing a known volume of the calibrator stock solution with a known volume of liquid not comprising D-dimers. As will be understood, calibrator samples may be diluted further to provide further calibrator samples having lower concentrations; thus, dilutions and serial dilutions may be used to provide the calibrator samples of the present invention in any arbitrary combination. According to the present invention, at least two calibrator samples are provided. As will be understood, in particular in case a non-linear calibration curve shall be provided, further calibrator samples may be used. Thus, in an embodiment, at least three, at least four, or at least five calibrator samples are provided. In a further embodiment, of from two to ten calibrator samples are provided, in a further embodiment, of from three to eight calibrator samples are provided, in a further embodiment, of from four to seven calibrator samples are provided, in a further embodiment, five or six calibrator samples are provided. In an embodiment, one of the calibrator samples has the same D-dimer concentration as the calibrator stock solution, i.e. in an embodiment, a calibrator sample may be an undiluted calibration stock solution. Thus, in an embodiment, all or all but one calibrator samples are derived from said calibrator stock solution by dilution.

According to the present invention, a calibrator sample may have a calculated concentration value, the term "calculated concentration value" referring to a concentration value of D-dimers which can be obtained by the aforesaid calculations based on the predetermined concentration of D-Dimers in the calibrator stock solution. Thus, in an embodiment, a calculated concentration value is a concentration value attributed to a sample by taking into account the dilution factor applied while deriving the calibrator sample from the calibrator stock solution. In an embodiment, a calculated concentration value is attributed to each calibrator sample; in a further embodiment, a calculated concentration value is attributed only to those calibrator samples, to which no assigned concentration values are assigned. Thus, in an embodiment in which an assigned concentration value is assigned to all calibrator samples, no calculated concentration value may be attributed to any of said calibrator samples.

According to the invention, an assigned concentration value is assigned to at least one calibrator sample. The term "assigned concentration value", as used herein, relates to a concentration value attributed to a calibrator sample, wherein said assigned concentration value deviates as specified herein below from a calculated concentration value which might be attributed to said calibrator sample; accordingly, the expression "wherein said assigned concentration value assigned to at least one calibrator sample deviates from the calculated concentration value of said calibrator sample" is used for clarification only. As used herein, "an assigned concentration value deviates from the calculated concentration value" means that the assigned concentration value differs from the calculated concentration value, i.e. is outside the applicable range of technical precision of the calculated concentration value. As will be understood by the skilled person, e.g., a calculated concentration value of 1.5 µg/ml will indicate a value of from 1.45 µg/ml to 1.54 µg/ml and, accordingly, in this example the assigned concentration value would be lower than 1.45 µg/ml or higher than 1.54 µg/ml. In a further embodiment, an assigned concentration value is higher than the calculated concentration value +5% or is lower than the calculated concentration value -5%. In a further embodiment, an assigned concentration value is higher than the calculated concentration value +10% or is lower than the calculated concentration value -10%. In a further embodiment, an assigned concentration value is higher than the calculated concentration value +15% or is lower than the calculated concentration value -15%. In a further embodiment, an assigned concentration value is higher than the calculated concentration value +20% or is lower than the calculated concentration value -20%. In a further embodiment, an assigned concentration value is higher than the calculated concentration value +50 % or is lower than the calculated concentration value -50 %. In a further embodiment, an assigned concentration value is higher than two times the calculated concentration value or is lower than half the calculated concentration value. In an embodiment, assigned concentration values are assigned to more than one calibrator sample. Thus, in an embodiment, assigned concentration values are assigned to at least two, in an embodiment to at least three, in a further embodiment to at least four, in a further embodiment to all calibrator samples. According to the invention, each assigned concentration value of a calibrator sample deviates from the calculated concentration value of the respective sample as specified above. As will be appreciated, the direction and/or the degree of deviation may vary between calibrator samples to which an assigned concentration value is assigned; e.g. in an embodiment, the assigned concentration value of a first calibrator sample may be less than the calculated concentration of said first calibrator sample -10 %, and the assigned concentration value of a second calibrator sample may be more than the calculated concentration of said second calibrator sample + 20 %. In an embodiment, the degree of deviation varies between calibrator samples to which an assigned concentration value is assigned. In a further embodiment, only the degree of deviation varies between calibrator samples to which an assigned concentration value is assigned, i.e. the direction of deviation (i.e. an assigned concentration value being higher or lower than the calculated concentration value) does not vary. Thus, in an embodiment, all assigned concentration values are higher than the respective calculated concentration values, or all assigned concentration values are lower than the respective calculated concentration values. In an embodiment, the at least one calibrator sample to which an assigned concentration value deviating from the calculated concentration value is assigned (i) is the calibrator stock solution and/or (ii) is derived from the calibrator stock solution by dilution. The assigned concentration value may be assigned manually, e.g. by adjusting calibration points for a calibration curve established manually, or by inputting said assigned concentration values manually into a device establishing a calibration curve; or the assigned concentration value may be assigned in an automated fashion, e.g. by reading a barcode into an appropriately equipped device, said device comprising a database comprising barcodes and associated assigned concentration values.

Thus, according to the present invention, a calibrator sample may have attributed (i) a calculated concentration value, (ii) an assigned concentration value, or (iii) calculated concentration value and an assigned concentration value. In an embodiment, in case an assigned concentration value is attributed to a sample, said assigned concentration value is used in establishing a calibration curve.

In an embodiment, assigned concentration values are obtained by comparing a calibration curve obtained with a specific method, e.g. an immunoturbidimetric method, to a reference calibration curve obtained with a different method, e.g. the method of Adema & Gebert as specified herein above and determining which concentration values have to be assigned to one or more calibrator sample(s) in order to obtain a calibration curve corresponding to, in an embodiment being essentially identical to, the reference calibration curve. In an embodiment, assigned concentration values are obtained by comparing a calibration curve obtained with a specific method on a specific device or device model to a reference calibration curve obtained with said specific method on a different device or device model and determining which concentration values have to be assigned to one or more calibrator sample(s) in order to obtain a calibration curve corresponding to, in an embodiment being essentially identical to, the reference calibration curve. As used herein, the term "reference calibration curve" relates to a calibration curve obtained by determining the values of an optical property in samples having pre-determined and non-identical concentrations of D-dimers, e.g. in selected plasma pools known to contain a certain amount of D-dimer. In an embodiment, a reference calibration curve is obtained in a reference measurement using non-diluted reference samples. In an embodiment, assigned concentration values are obtained by determining the values of an optical property for at least three samples having pre-determined and non-identical concentrations of D-dimers in said D-dimer assay. In an embodiment, at least four, in a further embodiment at least five of said samples are used.

In an embodiment, the reference calibration curve and the calibration curve are obtained using the same detection agent batch (lot) and/or the same batch (lot) of calibrator stock solution. As used herein, the terms "batch" and "lot", in an embodiment, relate to an entirety of reagent preparations of a specific kind produced in the same production process. Thus, in an embodiment, the calibrator stock solution and/or the at least one assigned concentration value obtained are allocated to one or more reagent lots. In an embodiment, allocating a calibrator stock solution to a reagent lot relates to reserving said calibrator stock solution for use with said reagent lot. In an embodiment, said calibrator stock solution is reserved for use with at least one reagent lot; in a further embodiment, said calibrator stock solution is reserved for use with more than one reagent lot; in a further embodiment, said calibrator stock solution is reserved for use with a multitude of reagent lots, in particular with at least two, in an embodiment at least five, in a further embodiment at least ten reagent lots. In an embodiment, allocating an assigned concentration value to a reagent lot relates to indicating that said assigned concentration value is applicable only if said reagent lot is used in the D-dimer assay. In an embodiment, the calibrator stock solution and/or the at least one assigned concentration value obtained are allocated to the specific type of device, in particular a device model, used in obtaining said assigned concentration value(s). Thus, in an embodiment, the assigned calibration values are determined in a lot-specific and device-specific manner. Accordingly, in an embodiment, the calibrator stock solution is associated with an identifier, e.g. a reference number or a barcode, which may be provided with the calibrator stock solution, e.g. by printing it onto the housing of the calibrator stock solution or by including it in the packaging thereof, said identifier providing information which reagent lot it shall be used with. In an embodiment the calibrator stock solution is associated with an identifier which may be provided on a leaflet or package insert accompanying said calibrator stock solution.

The term "optical property", as used herein, relates to a property which can be detected by an optical instrument, i.e. an instrument detecting photons. Specifically, the optical property may be or may comprise at least one property selected from the group consisting of: a reflection property, a transmission property, an emission property, a scattering property, a fluorescence property, a phosphorescence property, a diffraction property, and a polarization property. It will be understood that a change of at least one optical property as used herein encompasses the detection of the presence of a property which was not detectable before, the detection of the absence of a property which has been detected before, and the detection of quantitative changes of a property, i.e., the detection of the change of the signal strength which correlates to the extent of the change of the at least one optical property. In an embodiment, an optical property as referred to herein refers to a turbidity and/or an optical density of a sample, or properties associated therewith. In an embodiment, the optical property is an a parameter proportional to the ratio of radiant flux received by a sample to the radiant flux transmitted by said sample, e.g. an absorbance; in a further embodiment, the optical property determined is the ratio of absorbance and length of the light path through the sample, i.e. is an optical density. Methods of converting the optical property as defined above into a physical signal which can be read as a measurement value are well known in the art.

The term "calibration curve" is known to the skilled person, in particular in the context of biological and/or clinical assays. In an embodiment, the calibration curve is a graphical and/or mathematical representation of a relationship obtained in a calibration between values determined in a D-dimer assay, e.g. a values of an optical property, and the quantity of an analyte, e.g. the calculated or assigned concentration of a D-dimer. In an embodiment, the calibration curve is a straight or essentially straight line. In a further embodiment, the calibration curve is non-linear, i.e. is a curve in a strict sense. In a further embodiment, the calibration curve has at least one inflection point, in a further embodiment has exactly one inflection point. Thus, in an embodiment, the calibration curve is a sigmoidal calibration curve, wherein, in an embodiment, the mathematical sign of the slope of the calibration curve does not change over the calibration interval, and, in a further embodiment, the slope of the calibration curve is not zero over the whole calibration interval. According to the invention, for establishing a calibration curve, calculated concentration values are used for samples to which no assigned concentration value was assigned, and assigned concentration values are used for samples to which assigned concentration values were assigned. Accordingly, the calibration curve according to the present invention deviates in at least one optical property/concentration value pair from a calibration curve obtained from calculated concentration values only. As will be understood by the skilled person, a calibration curve may be established manually, assisted by automated equipment, or may be established by automated equipment, in particular a data processing device.

Advantageously, it was found in the work underlying the present invention that batch-to-batch and/or device model-to-model variations in measurements obtained in D-dimer assays can be compensated by readjusting the concentration values of calibrator samples to deviate from their calculated concentrations. By doing so, it is possible to obtain calibration curves allowing high-precision measurements even in case only one calibration solution is provided, from which further calibrator samples are derived by dilution. Moreover, high inter-device model reproducibility throughout the whole measuring range of a D-dimer assay can be ensured.

The definitions made above apply mutatis mutandis to the following. Additional definitions and explanations made further below also apply for all embodiments described in this specification mutatis mutandis.

The present invention further relates to a method for determining the D-dimer concentration according to claim 8; further disclosure relates to a method for determining the D-dimer concentration in a sample, comprising
a) contacting said sample with a detection agent specifically binding to D-dimers,
b) determining a value of an optical property of the sample/detection agent mixture of a),
c) providing a calibration curve according the present invention,
d) determining the D-dimer concentration in said sample based on the value of said optical property of the sample/detection agent mixture determined in b) and said calibration curve of c).

The method for determining the D-dimer concentration of the present invention is an in vitro method and may comprise further steps to those specifically mentioned. Further steps may, in embodiments, relate to providing a sample for step a), to preprocessing a sample, e.g. by centrifugation, and/or to contacting said sample to further agents, e.g. agents specifically recognizing the detection agent specifically binding to D-dimers, after step a).

The term "sample", as used herein, relates to a sample suspected or known to comprise D-dimers assessed by the present invention. In an embodiment, the sample is a sample of a subject suspected to suffer from thrombosis, in particular deep vein thrombosis, and/or from embolism, in particular lung embolism, and/ or is a sample from a pregnant subject, from a subject post partum (after having given birth), and/or from a subject showing up or being treated in an intensive care unit. In an embodiment, the sample is from a subject undergoing routine measurement as part of a regular health check-up. In an embodiment, the sample is or comprises a sample of a body fluid, a sample from a tissue or an organ, or a sample of wash/rinse fluid or a swab or smear obtained from an outer or inner body surface. In an embodiment, samples of stool, urine, saliva, cerebrospinal fluid, blood, serum, plasma, or lacrimal fluid are encompassed as samples by the method of the present invention. Samples can be obtained by use of brushes, (cotton) swabs, spatula, rinse/wash fluids, punch biopsy devices, puncture of cavities with needles or lancets, or by surgical instrumentation. However, samples obtained by well-known techniques including, in an embodiment, scrapes, swabs or biopsies are also included as samples used in the present invention. Cell-free fluids may be obtained from the body fluids or the tissues or organs by lysing techniques such as homogenization and/or by separating techniques such as filtration or centrifugation. In an embodiment, samples are obtained from body fluids known to comprise D-dimers assessed by the present invention, i.e., in an embodiment, samples are blood, plasma, serum, saliva, or the like. It is to be understood that a sample may be further processed in order to carry out the method of the present invention. Particularly, cells may be removed from the sample by methods and means known in the art. In an embodiment, the sample is a blood, serum, or plasma sample, in a further embodiment, a plasma sample, in particular a citrate plasma sample.

The term "subject", as used herein, relates to an animal, in an embodiment a mammal, in a further embodiment a primate, in a further embodiment a human. In an embodiment, the subject according to the present invention is a subject suspected to have an elevated level of D-dimers, in particular in a body fluid, especially in the blood. Thus, in an embodiment, the subject is suspected or known to suffer from thrombosis, embolism, coagulopathy, or a further condition causing blood D-dimer concentrations to increase. In an embodiment, the subject is suspected or known to suffer from lung embolism and/or deep vein thrombosis and/or is a pregnant subject, a subject post partum (after having given birth), and/or a subject showing up or being treated in an intensive care unit. In a further embodiment, the subject is a subject showing symptoms of lung embolism and/or deep vein thrombosis, for whom diagnosis of lung embolism and/or deep vein thrombosis shall be excluded.

The term "contacting", as used in the context of the methods of the present invention, is understood by the skilled person. In an embodiment, the term relates to bringing a compound used in the present invention, in particular a detection agent specifically binding to D-dimers, in physical contact with a sample, thereby allowing the detection agent and the sample to interact. In an embodiment, contacting includes additionally diluting the sample, e.g. with an appropriate buffer.

In the method for determining the D-dimer concentration, an optical property as specified elsewhere herein of the sample/detection agent mixture is determined. In an embodiment, the method is an immuno-photometric method; in a further embodiment, the method is an immunoturbidimetric method, i.e. is a method measuring an increase of turbidity of a sample after addition of a detection agent. Thus, in an embodiment, the method is a method determining absorbance and/or optical density of a sample/detection agent mixture as specified herein above.

Determining the D-dimer concentration in a sample based on the value of an optical property of the sample/detection agent mixture and a calibration curve of the present invention may be accomplished by any means deemed appropriate by the skilled person. E.g., in an embodiment, the D-dimer concentration may be determined by establishing a graphical representation of the calibration curve and by graphically determining the D-dimer concentration corresponding to the measured value of the optical property. In a further embodiment, the calibration curve is represented as a mathematical equation and the D-dimer concentration is calculated by applying the measured value of the optical property to said mathematical equation.

The present disclosure also relates to a kit for determining the D-dimer concentration in a sample, said kit comprising a calibrator stock solution having a predetermined concentration of D-dimers and a data carrier comprising at least one assigned concentration value for a calibrator sample.

The term "kit", as used herein, refers to a collection of the aforementioned compounds, means or reagents of the present disclosure which may or may not be packaged together. The components of the kit may be comprised by separate housings (i.e. as a kit of separate parts) or, in particular the binding agents, two or more components may be provided in a single housing. Moreover, it is to be understood that the kit of the present disclosure, in an embodiment, is to be used for practicing the methods referred to herein above. It is, in an embodiment, envisaged that components, in an embodiment all components, are provided in a ready-to-use manner for practicing the methods referred to above. In an embodiment, all or some of said components are provided in dried, such as in lyophilized form, wherein the component is reconstituted using a liquid such as an aqueous buffered solution. In an embodiment all or some of said components are provided in concentrated liquid form wherein the concentrated component is diluted using a liquid such as an aqueous buffered solution. Further, the kit, in an embodiment, contains instructions for carrying out said methods and, if applicable, said reconstitution of dried reagents. The instructions can be provided by a user's manual in paper- or electronic form. In addition, the manual may comprise instructions for interpreting the results obtained when carrying out the aforementioned methods using the kit of the present disclosure, and or an assigned concentration value as specified herein below. In an embodiment, the kit further comprises at least one detection agent specifically recognizing D-dimers. In a further embodiment, the at least one detection agent specifically recognizing D-dimers in the kit is covalently coupled to latex beads, in an embodiment latex particles of uniform size, in an embodiment latex particles of different size; or the kit comprises latex beads and means for coupling the detection agent to said latex beads. Also in an embodiment, the kit further comprises a further means for performing a D-dimer assay, e.g. a buffer.

As used herein, the term "data carrier comprising at least one assigned concentration value" is used in a wide sense and includes a data carrier physically including an assigned concentration, as well as a data carrier comprising information making an assigned concentration value determinable. Also, the data carrier comprising at least one assigned concentration value may be human readable and/or machine readable. Accordingly, the data carrier comprising at least one assigned concentration value may be a printed information indicating an applicable assigned concentration and an indication of the calibration sample to which said assigned concentration value is applicable for human reading, e.g. by including said information in a manual. However, the data carrier comprising at least one assigned concentration value may, in an embodiment, also be a, human- and/or machine-readable, identifier, e.g. a barcode or an RFID device; said identifier, in an embodiment, identifies a batch of calibrator stock solution, making possible identification of an applicable assigned concentration value and of the calibration sample to which said assigned concentration value is applicable in an appropriate database. In an embodiment, the same or a further identifier comprised in the kit allocates said calibrator stock solution and/or said data carrier comprising at least one assigned concentration value to one or more reagent lots, in particular a lot of a detection agent specifically recognizing D-dimers. Thus, in an embodiment, the calibrator stock solution is comprised in a housing comprising an identifier, said identifier allocating said calibrator stock solution and/or said data carrier to one or more reagent lots, in particular a lot of a detection agent specifically recognizing D-dimers. In a further embodiment, the same or a further identifier comprised in the kit allocates said calibrator stock solution and/or said data carrier comprising at least one assigned concentration value to one or more device model(s).

The present disclosure further relates to a device for determining the D-dimer concentration in a sample comprising means for detecting an optical property of a sample and a machine-readable data carrier comprising a database comprising at least one assigned concentration value for a predefined calibration sample for a D-dimer assay. As specified elsewhere herein, the assigned concentration value assigned to at least one calibrator sample deviates from the calculated concentration value of said calibrator sample

The term "device", as used herein, relates to a system of means comprising at least the aforementioned means operatively linked to each other as to allow the result of the determination to be obtained. Preferred means for contacting a sample with a detection agent are disclosed above in connection with the methods of the invention. How to link the means in an operating manner will depend on the type of means included into the device. In an embodiment, the means are comprised by a single device.

In an embodiment, the sample treatment unit comprises a receptacle for a sample. The receptacle may directly contact the sample, or may be a receptacle for a further means receiving the sample, wherein the further means may be e.g. a multi-well plate, to which a sample or a multiplicity of samples may be applied. Moreover, the sample treatment unit, in an embodiment, comprises a calibrator stock solution, e.g. in a reservoir connected to a dosing means, e.g. a tubing connected to a pump. In a further embodiment, the device comprises one calibrator stock solution having a predetermined D-dimer concentration; thus, in an embodiment, the device comprises only one, i.e. exactly one, calibrator stock solution. In an embodiment, the sample treatment unit further comprises at least one detector compound, e.g. in a dried form or in a reservoir connected to a dosing means, e.g. a tubing connected to a pump. In a further embodiment, the sample treatment unit comprises means for mixing and means for adjusting the temperature of a reaction mixture.

In an embodiment, the result of the determination is obtained by performing a detection measurement on an appropriate detection unit. Thus, in an embodiment, the analyzing unit of the device of the present disclosure further comprises a detection unit for detecting an optical property of a sample and of a sample/detection agent mixture. Means suitable as a detection unit according to the present disclosure are known to the skilled person and include, e.g. photometric devices.

The device of the present disclosure further comprises a machine-readable data carrier comprising a database comprising at least one assigned concentration value for a predefined calibration sample for a D-dimer assay. In an embodiment, said machine-readable data carrier is operatively connected to an evaluation unit or an evaluation device.

In an embodiment, the device of the present disclosure further comprises an evaluation unit or is part of an analytic system, said analytic system further comprising an evaluation device. As will be understood by the skilled person, the evaluation means may be comprised in the same housing as the device of the disclosure as an evaluation unit, or may be a separate device, i.e. an evaluation device. In an embodiment, the evaluation unit or device comprises a microprocessor programmed to receive output data from an output unit of the analyzing unit of the present disclosure and to perform logical operations providing an evaluation of said output data. Evaluation of output data may comprise, e.g., correcting data for values measured in one or more control detection reaction, statistical calculations, e.g. calculating means of two or more parallel detection reactions, providing calculated concentration values, e.g. by applying dilution factors to the concentration of the calibrator stock solution, comparing output data to reference values, compiling data in a list, and the like. In an embodiment, the evaluation device further comprises a data storage unit. In a further embodiment, said data storage unit comprises reference values, e.g. in a reference value data base. Moreover, in an embodiment, the data storage unit is adapted to store output data received from a device of the present disclosure, as specified above. In an embodiment, the evaluation unit or device comprises means for establishing a calibration curve for a D-dimer assay based on values of an optical property determined by the analysis unit, on the assigned concentration value or the assigned concentration values provided on the machine-readable data carrier of the device, and on calculated concentration values for calibrator samples to which no assigned concentration value was assigned, provided by the evaluation unit or device. In a further embodiment, the evaluation unit or device comprises means for determining the D-dimer concentration in a sample based on the value of an optical property of a sample/detection agent mixture determined by the analysis unit and the calibration curve provided by the evaluation unit or device as specified above.

In an embodiment, the device of the present disclosure further comprises a data output unit, connected to the detection unit and/or to the evaluation unit or device. The data output unit, in an embodiment, is adapted to output data obtained by the detection unit or generated by the evaluation unit or device. Suitable data output units are known to the skilled person and include simple output units such as an indicator lamp or a display indicating that an increased D-dimer concentration was detected. An output unit may, however, also be an interface to an output device, wherein said interface may be any kind of means of transferring data, including, e.g. cable connections like USB, wireless connections like wireless LAN, bluetooth, and the like, or indirect connections such as data transfer by instant messaging, email, or the like.

In an embodiment, where means for automatically detecting a D-dimer concentration are applied, the data obtained by said automatically operating means can be processed by, e.g., a computer program in order to establish or aid in establishing a diagnosis (e.g. identifying a subject not suffering from thrombosis and not suffering from embolism). Typical means for detection are disclosed in connection with embodiments relating to the methods of the invention above. In such a case, the means are operatively linked in that the user of the system brings together the result of the determination of the amount and the diagnostic value thereof due to the instructions and interpretations given in a manual. The person skilled in the art will realize how to link the means without further inventive skills. Typical devices are those which can be applied without the particular knowledge of a specialized clinician, e.g., electronic devices which merely require loading with a sample. The results may be given as output of parametric diagnostic raw data, in embodiments, as absolute or relative amounts. It is to be understood that these data will need interpretation by the clinician. However, also envisaged are expert system devices wherein the output comprises processed diagnostic raw data the interpretation of which does not require a specialized clinician.

The present disclosure further relates to a use of at least one calibration sample having an assigned concentration value for determining the D-dimer concentration in a sample. As will be understood from the above, said use, in an embodiment, also includes the use of at least two calibration samples having an assigned concentration value, in a further embodiment of at least three calibration samples having an assigned concentration value, in a further embodiment of at least four calibration samples having an assigned concentration value, in a further embodiment of at least five calibration samples having an assigned concentration value, for determining the D-dimer concentration in a sample.

The present disclosure also relates to a database comprising at least one assigned concentration value for a predefined calibration sample. In an embodiment, said database further comprises an identifier for at least one lot of a composition comprising a detection agent specifically recognizing D-dimers. Moreover, the present disclosure relates to a data carrier comprising the database of the present disclosure.

The disclosure further discloses and proposes a computer program including computer-executable instructions for performing the method according to the present invention in one or more of the embodiments enclosed herein when the program is executed on an analytic device, computer or computer network. Specifically, the computer program may be stored on a computer-readable data carrier. Thus, specifically, one, more than one or even all of the method steps as indicated above may be performed by using a computer or a computer network, preferably by using a computer program.

The disclosure further discloses and proposes a computer program product having program code means, in order to perform the method according to the present invention in one or more of the embodiments enclosed herein when the program is executed on an analytic device, computer or computer network. Specifically, the program code means may be stored on a computer-readable data carrier.

Further, the disclosure discloses and proposes a data carrier having a data structure stored thereon, which, after loading into a computer or computer network, such as into a working memory or main memory of the computer or computer network, may execute the method according to one or more of the embodiments disclosed herein.

The disclosure further proposes and discloses a computer program product with program code means stored on a machine-readable carrier, in order to perform the method according to one or more of the embodiments disclosed herein, when the program is executed on a computer or computer network. As used herein, a computer program product refers to the program as a tradable product. The product may generally exist in an arbitrary format, such as in a paper format, or on a computer-readable data carrier. Specifically, the computer program product may be distributed over a data network.

Finally, the disclosure proposes and discloses a modulated data signal which contains instructions readable by a computer system or computer network, for performing the method according to one or more of the embodiments disclosed herein.

In an embodiment, referring to the computer-implemented aspects of the disclosure, one or more of the method steps or even all of the method steps of the method according to one or more of the embodiments disclosed herein may be performed by using a computer or computer network. Thus, generally, any of the method steps including provision and/or manipulation of data may be performed by using a computer or computer network. Generally, these method steps may include any of the method steps, typically except for method steps requiring manual work, such as providing the samples and/or certain aspects of performing the actual measurements.

Specifically, the present disclosure further discloses:
- A computer or computer network comprising at least one processor, wherein the processor is adapted to perform the method according to one of the embodiments described in this description,
- a computer loadable data structure that is adapted to perform the method according to one of the embodiments described in this description while the data structure is being executed on a computer,
- a computer program, wherein the computer program is adapted to perform the method according to one of the embodiments described in this description while the program is being executed on a computer,
- a computer program comprising program means for performing the method according to one of the embodiments described in this description while the computer program is being executed on a computer or on a computer network,
- a computer program comprising program means according to the preceding embodiment, wherein the program means are stored on a storage medium readable to a computer,
- a storage medium, wherein a data structure is stored on the storage medium and wherein the data structure is adapted to perform the method according to one of the embodiments described in this description after having been loaded into a main and/or working storage of a computer or of a computer network, and
- a computer program product having program code means, wherein the program code means can be stored or are stored on a storage medium, for performing the method according to one of the embodiments described in this description, if the program code means are executed on a computer or on a computer network.

### Figure Legend

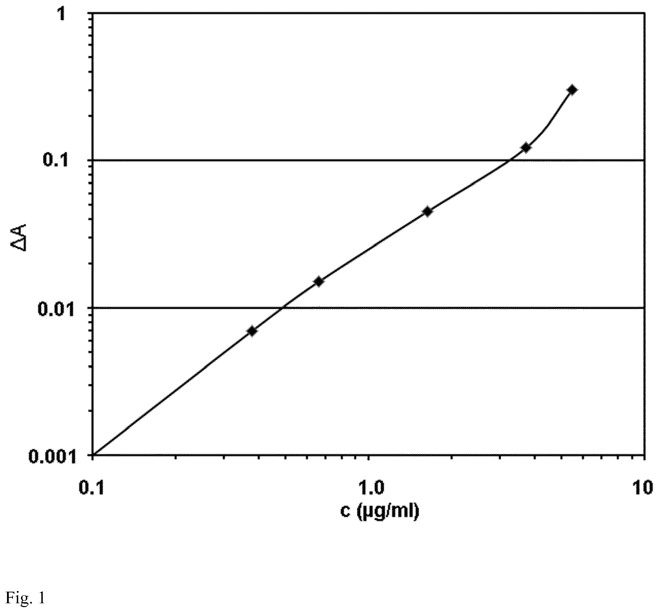

Fig. 1: Calibration curve obtained according to the method of the present invention, x-axis: D-dimer concentration in µg/ml; y-axis: change in Absorption (ΔA). The figure shows a typical calibration curve on the t411 instrument. The calibrator values of the stock solution and dilutions used to construct this calibration curve are determined according to the method described above. The data point with the highest d-dimer concentration is taken from a calibrator stock solution whereas the data points with the lower concentrations are derived from assigned values after automated dilution of the calibrator stock on the instrument

The following Example shall merely illustrate the invention. It shall not be construed, whatsoever, to limit the scope of the invention.

### Example 1: Calibration

To calibrate the D-Dimer assay on a cobas t 411 instrument, a calibrator stock solution was automatically diluted by the instrument in the ratios of 1:1; 1:2; 1:4; 1:8 and 1:16 to generate four further calibrator samples. A reagent lot specific target value (assigned concentration value) was assigned to each calibrator sample during a standardization procedure comprising the following steps:
1) The first step was to establish human plasma pools as secondary calibrators. The pools were generated with the goal to cover the primary measuring range of the D-Dimer assay on the target instrument where the standardization shall be performed, in this example Roche's cobas t 411. For this purpose, 8 plasma pools were generated using fresh human plasma. Primary target values (concentration values) of these plasma pools were obtained using an established clinical chemistry system, in this case Roche's cobas c501.
2) In a second step, the plasma pools were used to assign D-Dimer reagent lot specific target values (assigned concentrations) to various lots of D-Dimer calibrator stock solution. For this purpose, the plasma pools were measured on the cobas t 411 as specified in Example 2 and a method comparison (Pasing-Bablok) was calculated between the measured and the assigned values of the pools. The target values of the individual calibration set points on the cobas t 411 instrument were individually adjusted by fitting the method comparison curve as close as possible to the optimal regression line until a re-calculation of the method comparison allowed meeting most narrow specifications.

This procedure was carried out for each combination of D-Dimer reagent lot and D-Dimer calibrator stock solution to be used by the customer and allows for excellent comparability of the cobas t 411 to the clinical chemistry analyzer c 501.

### Example 2: Measurement

To run a D-Dimer test, commercially available D-Dimer reagent, D-Dimer calibrator solution or calibrator stock solution and two D-Dimer controls (all from Roche Diagnostics) were used.

To run one of Roche's D-Dimer assays, in general an aliquot of human plasma or serum is pipetted to a cuvette to contact the D-Dimer reagent with buffer R1, containing 250 mmol/L TRIS/HCl buffer. Subsequently, the reagent R2, containing latex particles coated with monoclonal anti-human D-Dimer, is added to this mixture to initiate an immuno-turbidimetric reaction. As an alternative, the immunoturbidimetric reaction can also be started by adding the sample to reagents R1 and R2. D-Dimers contained in the human sample are cross-linked by the antibodies coated on the latex beads, resulting in an agglutination of the beads. This leads to a change in absorption of the sample detected by the photometer unit of the instrument, and thus allows for the determination of the D-Dimer concentration in the tested sample in the unit [µg FEU/ml].

On the target instrument where the standardization shall be performed, in this example Roche's cobas t 411, 20 µl human plasma and 80 µl R1 reagent were diluted with 30 µl Imidazole buffer before 70 µl R2 reagent were added and the immuno-turbidimetric reaction took place.

## Claims

1. A method for providing a calibration curve for an optical D-dimer assay comprising
a) providing a calibrator stock solution having a predetermined D-dimer concentration,
b) providing at least two calibrator samples, wherein said at least two calibrator samples are derived from said calibrator stock solution, wherein all or all but one calibrator samples are derived from said calibrator stock solution by dilution,
c) determining the value of an optical property of said calibrator samples of b) in said D-dimer assay,
d) assigning an assigned concentration value to at least one calibrator sample of b), wherein said assigned concentration value assigned to at least one calibrator sample deviates from the calculated concentration value of said calibrator sample, wherein said calculated concentration value is a concentration value attributed to a calibrator sample by taking into account the dilution factor applied while deriving the calibrator sample from the calibrator stock solution; and wherein said assigned concentration value was obtained by comparing a calibration curve obtained with the method used in step c) to a reference calibration curve obtained for said calibrator stock solution with a different method, obtained on a different device, or obtained on a different device model, and
e) establishing a calibration curve based on the values of said optical property of c), on the assigned concentration value or the assigned concentration values of d), and on calculated concentration values for calibrator samples to which no assigned concentration value was assigned.

2. The method of claim 1, wherein at least three, in an embodiment at least four, in a further embodiment at least five calibrator samples are provided.

3. The method of claim 1 or 2, wherein all or all but one calibrator samples are derived from said calibrator stock solution by dilution.

4. The method of any one of claims 1 to 3, wherein an assigned concentration value is assigned to at least two, in an embodiment at least three, in a further embodiment at least four, calibrator samples.

5. The method of any one of claims 1 to 4, wherein said optical property is turbidity and/or optical density.

6. The method of any one of claims 1 to 5, wherein said D-dimer assay comprises contacting said calibrator samples with a detection agent specifically recognizing D-dimers.

7. The method of any one of claims 1 to 6, wherein said calibration curve is a non-linear calibration curve.

8. A method for determining the D-dimer concentration in a sample, comprising
a) contacting said sample with a detection agent specifically binding to D-dimers,
b) determining a value of an optical property of the sample/detection agent mixture of a),
c) providing a calibration curve according to any one of claims 1 to 7,
d) determining the D-dimer concentration in said sample based on the value of said optical property of the sample/detection agent mixture determined in b) and said calibration curve of c).

9. The method of claim 8, wherein said calibrator stock solution and/or said at least one assigned concentration value are allocated to one or more reagent lots, in particular a lot of said detection agent.

## Patentansprüche

1. Verfahren zum Bereitstellen einer Kalibrierungskurve für ein optisches D-Dimer-Assay, das Folgendes umfasst:
a) Bereitstellen einer Kalibratorstammlösung mit einer vorbestimmten D-Dimer-Konzentration,
b) Bereitstellen von wenigstens zwei Kalibratorproben, wobei die wenigstens zwei Kalibratorproben von der Kalibratorstammlösung abgeleitet sind, wobei alle oder alle außer einer Kalibratorproben durch Verdünnung von der Kalibratorstammlösung abgeleitet sind,
c) Bestimmen des Wertes einer optischen Eigenschaft der Kalibratorproben von b) in dem D-Dimer-Assay,
d) Zuweisen eines zugewiesenen Konzentrationswertes zu wenigstens einer Kalibratorprobe von b), wobei der zugewiesene Konzentrationswert, der wenigstens einer Kalibratorprobe zugewiesen ist, von dem berechneten Konzentrationswert der Kalibratorprobe abweicht, wobei der berechnete Konzentrationswert ein Konzentrationswert ist, der einer Kalibratorprobe zugeschrieben wird, indem der Verdünnungsfaktor berücksichtigt wird, der während des Ableitens der Kalibratorprobe von der Kalibratorstammlösung angewandt wird; und wobei der zugewiesene Konzentrationswert durch Vergleichen einer Kalibrierungskurve, die die mit dem in Schritt c) verwendeten Verfahren erhalten wurde, mit einer Referenzkalibrierungskurve erhalten wurde, die für die Kalibratorstammlösung mit einem anderen Verfahren erhalten wurde, auf einer anderen Vorrichtung erhalten wurde oder auf einem anderen Vorrichtungsmodell erhalten wurde, und
e) Einrichten einer Kalibrierungskurve basierend auf den Werten der optischen Eigenschaft aus c), auf dem zugewiesenen Konzentrationswert oder den zugewiesenen Konzentrationswerten von d) und auf berechneten Konzentrationswerten für Kalibratorproben, denen kein zugewiesener Konzentrationswert zugewiesen wurde.

2. Verfahren nach Anspruch 1, wobei wenigstens drei, bei einer Ausführungsform wenigstens vier, bei einer weiteren Ausführungsform wenigstens fünf Kalibratorproben bereitgestellt werden.

3. Verfahren nach Anspruch 1 oder 2, wobei alle oder alle außer einer Kalibratorprobe durch Verdünnung von der Kalibratorstammlösung abgeleitet werden.

4. Verfahren nach einem der Ansprüche 1 bis 3, wobei ein zugewiesener Konzentrationswert wenigstens zwei, bei einer Ausführungsform wenigstens drei, bei einer weiteren Ausführungsform wenigstens vier Kalibratorproben zugewiesen wird.

5. Verfahren nach einem der Ansprüche 1 bis 4, wobei die optische Eigenschaft eine Trübung und/oder eine optische Dichte ist.

6. Verfahren nach einem der Ansprüche 1 bis 5, wobei das D-Dimer-Assay Kontaktieren der Kalibratorproben mit einem Detektionsmittel, das speziell D-Dimere erkennt, umfasst.

7. Verfahren nach einem der Ansprüche 1 bis 6, wobei die Kalibrierungskurve eine nichtlineare Kalibrierungskurve ist.

8. Verfahren zum Bestimmen der D-Dimer-Konzentration in einer Probe, das Folgendes umfasst:
a) Kontaktieren der Probe mit einem Detektionsmittel, das speziell an D-Dimere bindet,
b) Bestimmen eines Wertes einer optischen Eigenschaft des Probe/Detektionsmittel-Gemischs aus a),
c) Bereitstellen einer Kalibrierungskurve nach einem der Ansprüche 1 bis 7,
d) Bestimmen der D-Dimer-Konzentration in der Probe basierend auf dem Wert der optischen Eigenschaft des Probe/Detektionsmittel-Gemischs, der in b) bestimmt wurde, und der Kalibrierungskurve aus c).

9. Verfahren nach Anspruch 8, wobei die Kalibratorstammlösung und/oder der wenigstens eine zugewiesene Konzentrationswert einer oder mehreren Reagenzchargen, insbesondere einer Charge des Detektionsmittels, zugeordnet werden.

## Revendications

1. Procédé pour fournir une courbe d'étalonnage pour un dosage optique de D-dimère comprenant :
a) la fourniture d'une solution mère étalon ayant une concentration prédéterminée en D-dimère,
b) la fourniture d'au moins deux échantillons étalons, lesdits au moins deux échantillons étalons étant dérivés de ladite solution mère étalon, tous les échantillons étalons ou tous les échantillons étalons sauf un étant dérivés de ladite solution mère étalon par dilution,
c) la détermination de la valeur d'une propriété optique desdits échantillons étalons de b) dans ledit dosage de D-dimère,
d) l'attribution d'une valeur de concentration attribuée à au moins un échantillon étalon de b), ladite valeur de concentration attribuée, attribuée à au moins un échantillon étalon s'écartant de la valeur de concentration calculée dudit échantillon étalon, ladite valeur de concentration calculée étant une valeur de concentration attribuée à un échantillon étalon en tenant compte du facteur de dilution appliqué lors de la dérivation de l'échantillon étalon à partir de la solution mère étalon ; et ladite valeur de concentration attribuée ayant été obtenue en comparant une courbe d'étalonnage obtenue par le procédé utilisé à l'étape c) à une courbe d'étalonnage de référence obtenue pour ladite solution mère étalon par un procédé différent, obtenue sur un dispositif différent, ou obtenue sur un modèle de dispositif différent, et
e) l'établissement d'une courbe d'étalonnage basée sur les valeurs de ladite propriété optique de c), sur la valeur de concentration attribuée ou les valeurs de concentration attribuées de d), et sur les valeurs de concentration calculées pour les échantillons étalons auxquels aucune valeur de concentration attribuée n'a été attribuée.

2. Procédé selon la revendication 1, au moins trois, au moins quatre dans un mode de réalisation, au moins cinq échantillons étalons dans une autre mode de réalisation étant fournis.

3. Procédé selon la revendication 1 ou 2, tous les échantillons étalons ou tous les échantillons étalons sauf un étant obtenus à partir de ladite solution mère étalon par dilution.

4. Procédé selon l'une quelconque des revendications 1 à 3, une valeur de concentration attribuée étant attribuée à au moins deux, au moins trois dans un mode de réalisation, au moins quatre échantillons étalons dans un autre mode de réalisation.

5. Procédé selon l'une quelconque des revendications 1 à 4, ladite propriété optique étant la turbidité et/ou la densité optique.

6. Procédé selon l'une quelconque des revendications 1 à 5, ledit dosage de D-dimère comprenant la mise en contact desdits échantillons étalons avec un agent de détection reconnaissant spécifiquement les D-dimères.

7. Procédé selon l'une quelconque des revendications 1 à 6, ladite courbe d'étalonnage étant une courbe d'étalonnage non linéaire.

8. Procédé pour déterminer la concentration en D-dimère dans un échantillon, comprenant :
a) la mise en contact dudit échantillon avec un agent de détection se liant spécifiquement aux D-dimères,
b) la détermination d'une valeur d'une propriété optique du mélange échantillon/agent de détection de a),
c) la fourniture d'une courbe d'étalonnage selon l'une quelconque des revendications 1 à 7,
d) la détermination de la concentration en D-dimère dans ledit échantillon sur la base de la valeur de ladite propriété optique du mélange échantillon/agent de détection déterminée en b) et de ladite courbe d'étalonnage de c).

9. Procédé selon la revendication 8, ladite solution mère étalon et/ou ladite au moins une valeur de concentration attribuée étant attribuées à un ou plusieurs lots de réactifs, en particulier un lot dudit agent de détection.
